# FASCICULE DE BREVET EUROPEEN

(11) **EP 0 252 854 B1**
(45) Date de publication et mention de la délivrance du brevet: **14.05.1997**
(21) Numéro de dépôt: 87401649.6
(22) Date de dépôt: 10.07.1987
(51) Int. Cl.: C12N 15/00, C12N 1/16, C12P 21/02, A61K 38/00

(54) **Bloc fonctionnel d'ADN et plasmide codant pour l'hirudine, levure transformée et procédé de préparation de l'hirudine.**
Funktioneller DNS-Block und für Hirudin kodierendes Plasmid, transformierte Hefe aud Verfahren zur Herstellung von Hirudin.
Functional DNA block and plasmid coding for hirudine, transformed yeast and process for the preparation of hirudine.

(30) Priorité: 10.07.1986 FR 8610090; 01.12.1986 FR 8616722
(43) Date de publication de la demande: 13.01.1988
(73) Titulaire: Behringwerke Aktiengesellschaft, 35041 Marburg (DE)
(72) Inventeur: Labat, Nathalie, F-67000 Strasbourg (FR); Loison, Gérard, F-67000 Strasbourg (FR); Balland, Alain, F-67370 Truchtersheim (FR); Lemoine, Yves, F-67100 Strasbourg-Neudorf (FR)
(74) Mandataire: Warcoin, Jacques

(56) Documents cités:
- EP-A- 0 158 564
- EP-A- 0 168 342
- EP-A- 0 171 024
- EP-A- 0 200 655
- WO-A-86/03517
- PROCEEDINGS OF THE NATIONAL ACADEMY OF SCIENCES OF THE USA, vol. 83, no. 4, février 1986, pages 1084-1088, Washington, US; R.P. HARVEY et al.: "Cloning and expression of a cDNA coding for the anticoagulant hirudin from the bloodsucking leech, Hirudo medicinalis"

## Description

La présente demande concerne des perfectionnements apportés à la demande de brevet français n^{o} 85 06672. Elle concerne des vecteurs d'expression d'une protéine ayant l'activité de l'hirudine de sangsue (H. medicinalis).

La demande de brevet n^{o} 85 06672 décrit un procédé permettant de faire produire par des levures telles que S. cerevisiae de l'hirudine sécrétée dans le milieu de culture sous forme active.

La présente invention concerne de nouveaux vecteurs qui assurent la production de l'hirudine, notamment HV1, dans de meilleures conditions.

Plus particulièrement, la présente invention concerne des blocs fonctionnels d'ADN permettant la préparation de l'hirudine à partir de levures, caractérisés en ce qu'ils comportent au moins la séquence :

- Sₜᵣ - Lₑₓ - S_{cl} - gène H

. gène H étant le gène codant pour l'hirudine ou l'un de ses variants ; le gène H est, de préférence, selon la présente invention le gène codant pour HV1 ou HV2 ; éventuellement, le gène H peut être suivi d'une séquence de terminateur de levure, par exemple celui du gène PGK ;
. Sₜᵣ est une séquence d'ADN comportant les signaux assurant la transcription du gène H par la levure ;
. Lₑₓ est une séquence leader nécessaire pour obtenir l'excrétion du produit du gène ;
. S_{cl} est une séquence d'ADN codant pour le site de clivage par la protéinase yscF, site de clivage qui est unique et situé dans le précurseur de l'hirudine immédiatement en amont du gène H ; en outre, l'élément S_{cl} - gène H peut être répété plusieurs fois.

Ce type de bloc d'expression permet d'obtenir une protéine unique, mature et correcte.

En effet, avec la construction décrite dans la demande de brevet n^{o} 85 06672, S. cerevisiae produit l'hirudine sous forme d'un précurseur présentant deux sites de coupure pour la protéinase yscF, produit du gène KEX2, (1). L'activité endo-peptidase de yscF est nécessaire à la maturation de ce précurseur de l'hirudine . Cependant, seuls les évènements de coupure au deuxième site, celui le plus éloigné de l'extrémité NH₂ du précurseur, permet la libération de l'hirudine active ; par contre, lorsque la coupure a lieu au premier site et non au deuxième, c'est une forme plus longue qui est sécrétée et qui diffère de l'hirudine par une extension de résidus aminoacides à l'extrémité NH₂. Cette dernière forme non désirable peut contaminer l'hirudine mature et correcte au cours de différentes étapes de purification et de ce fait compliquer le procédé de purification et entraîner une baisse de rendement dans la préparation d'hirudine pure.

Une autre construction décrite dans EP 200 655 comprend un unique site de clivage Lys - Arg situé immédiatement en amont du gène de l'hirudine avec le promoteur du gène MF alpha 1

Par la présente invention, on fait synthétiser à la levure un précurseur de l'hirudine qui ne possède qu'un seul site de coupure pour yscF de façon à ce que sa maturation en hirudine ne conduise à sécréter que la forme recherchée.

Parmi les séquences Lₑₓ, il faut citer en particulier la séquence dite "pre-pro" qui permet l'excrétion de la phéromone sexuelle alpha de levure.

On a choisi comme exemple de système de sécrétion, celui de la phéromone alpha, c'est-à-dire que, dans la séquence précédente, la séquence Lₑₓ provient du gène de la phéromone sexuelle alpha de levure, mais d'autres systèmes pourraient être utilisés (par exemple le système de la protéine Killer) (4).

De façon préférentielle, il est intéressant que les levures hôtes des plasmides portant les blocs fonctionnels selon l'invention puissent être d'un type sexuel quelconque et puissent, en outre, être cultivées en absence de une pression de sélection.

Dans ces conditions, les levures transformées peuvent être mises en oeuvre au stade industriel de façon particulièrement commode.

Pour ce faire, la présente invention propose des blocs d'expression d'ADN tels que décrits précédemment mais dans lesquels la séquence Sₜᵣ comporte un promoteur d'un gène de levure différent du promoteur du gène MFalphal, il peut s'agir, par exemple, du promoteur du gène PGK de la levure. La présente invention a donc pour objet un bloc d'expression tel que revendiqué dans les revendications 1 à 5.

En effet, dans les constructions décrites dans la demande de brevet n^{o} 85 06672, le gène du précurseur de l'hirudine est transcrit à partir du promoteur du gène MFalphal. Ce promoteur fonctionne au maximum de sa puissance dans les souches de type sexuel Matalpha. Le choix de l'hôte se restreint donc à ce type de souche, ce qui exclut les souches de type sexuel Mata ainsi que celles diploïdes ou polyploïdes dépourvues de type sexuel. En particulier, cela exclut les souches de levure utilisées dans l'industrie qui rentrent le plus souvent dans cette dernière catégorie. En changeant de promoteur, on peut obtenir une expression de l'hirudine indépendante du signe sexuel de la souche hôte.

De façon générale, ces blocs d'ADN seront portés par des plasmides comportant, en outre, une origine de réplication dans les levures, par exemple l'origine de réplication du plasmide 2µ.

Pour faciliter la construction des vecteurs, ces plasmides pourront être des "navettes coli-levure" et comporter des éléments qui permettent leur réplication dans E. coli, par exemple une origine de réplication telle que celle de pBR322 et un marqueur de sélection comme amp^{R}. Ces plasmides comporteront également un marqueur de sélection pour la levure, par exemple un gène codant pour la résistance à certains composés chimiques toxiques ou un gène permettant de complémenter une auxotrophie ; on utilisera plus particulièrement le gène URA3.

En effet, on a montré dans le brevet WO 86/01224 que l'introduction de certaines mutations conférant la résistance au 5-FU dans une souche ura3 transformée en ura+ par le biais d'un plasmide permettait un plus grand choix de milieux pour la culture de ces souches transformées ; on pourrait en particulier utiliser les milieux complexes industriels.

La présente invention concerne plus particulièrement la synthèse et la sécrétion du variant HV1.

Le gène HV1 a déjà été synthétisé chimiquement en vue de son expression dans E. coli (brevet français n^{o} 84 13250).

La plupart des oligonucléotides synthétiques utilisés dans le brevet n^{o} 84 13250 ont été conservés pour la construction de la séquence codant pour le précurseur d'HV1, à l'exception de ceux qui permettent les différentes jonctions avec les séquences qui seront fusionnées en amont et en aval de la séquence.

L'obtention d'une hirudine sécrétée nécessite la présence d'un précurseur contenant les informations nécessaires à la sécrétion et à la maturation correcte de ce polypeptide suivant le chemin métabolique normal de la levure. Dans la demande de brevet n^{o} 85 06672, on a montré que la séquence NH₂-terminale du précurseur du facteur alpha pouvait être utilisée pour la sécrétion du variant HV2 de l'hirudine. La présente invention concerne l'application de ce système à la sécrétion du variant HV1.

L'invention concerne en particulier un bloc fonctionnel d'ADN permettant la préparation de l'hirudine à partir de levure, caractérisé en ce qu'il comporte au moins :
. le gène de HV1 ;
. une séquence d'ADN (Sₜᵣ) comportant les signaux assurant la transcription du gène de HV1 par la levure.

Ce bloc fonctionnel, intégré dans un plasmide ou dans les chromosomes d'une levure, de préférence du genre Saccharomyces, pourra permettre, après transformation de ladite levure, l'expression de l'hirudine, soit sous forme active, soit sous forme d'un précurseur inactif, pouvant régénérer l'hirudine par activation.

Ces blocs fonctionnels présentent, de préférence la structure suivante :

- Sₜᵣ - Lₑₓ - S_{cl} - gène H -

. gène H étant le gène codant pour HV1 ; le gène H est, le cas échéant, suivi d'une séquence de terminateur de levure telle que celle du gène PGK ;
. Sₜᵣ est une séquence d'ADN comportant les signaux assurant la transcription du gène H par la levure ;
. Lₑₓ est une séquence leader nécessaire pour obtenir l'excrétion du produit du gène ;
. S_{cl} est une séquence d'ADN codant pour un site de clivage comportant, le cas échéant, à l'extrémité 3', avant le gène H, un codon ATG ou deux codons codant pour Lys-Arg ou cinq codons codant pour Ser-Leu-Asp-Lys-Arg ;
. en outre, l'élément S_{cl}-gène H peut être répété plusieurs fois.

Parmi les séquences Lₑₓ, il faut citer celle de la phéromone sexuelle alpha de levure, et pour Sₜᵣ, il faut citer le promoteur du gène MFalphal et également le promoteur du gène PGK, mais d'autres séquences peuvent être utilisées, en particulier on a choisi comme exemple de système de sécrétion celui de la phéromone alpha, c'est-à-dire que, dans la séquence précédente, la séquence Lₑₓ provient du gène de la phéromone sexuelle alpha de levure, mais d'autres systèmes pourraient être utilisés (par exemple le système de la protéine Killer) (4).

Pour diriger l'expression et la sécrétion de l'hirudine dans le milieu de culture, le gène correspondant est intégré dans un vecteur de levure ou un plasmide qui comprend le bloc fonctionnel décrit ci-dessus et au moins une origine de réplication dans les levures. Le plasmide comporte de préférence les éléments suivants :
. l'origine de réplication du plasmide de levure 2µ,
. le gène ura3,
. une origine de réplication dans E. coli et un marqueur de résistance à un antibiotique,
. la région 5' du gène MFalphal qui comprend le promoteur de transcription, la séquence "leader" et la séquence pre-pro du précurseur du facteur alpha, cette séquence sera fusionnée, en phase, en amont de la séquence codante de l'hirudine HV1,
. le terminateur de transcription du gène PGK de la levure qui sera placé en aval du gène de l'hirudine.

De façon générale, les blocs d'expression selon l'invention pourront être intégrés dans une levure, en particulier Saccharomyces, soit dans un plasmide à réplication autonome, soit dans le chromosome de la levure.

Lorsque le plasmide est autonome, il comportera des éléments assurant sa réplication, c'est-à-dire une origine de réplication telle que celle du plasmide 2µ. En outre, le plasmide pourra comporter des éléments de sélection tels que le gène URA3 ou LEU2 qui assurent la complémentation de levures ura3⁻ ou leu2⁻. Ces plasmides peuvent également comporter des éléments assurant leur réplication dans les bactéries, lorsque le plasmide doit être un plasmide "navette", par exemple une origine de réplication telle que celle de pBR322, un gène marqueur tel que Amp^{r} et/ou d'autres éléments connus de l'homme de métier.

La présente invention concerne également les levures transformées par un bloc d'expression selon l'invention, soit porté par un plasmide, soit intégré dans ses chromosomes, en particulier les souches de Saccharomyces et spécialement les souches de S. cerevisiae notamment de type sexuel Mata ou bien les souches diploïdes ou polyploïdes dépourvues de type sexuel, mais surtout les souches de levure ura⁻ transformées ura+ par un plasmide selon l'invention et devenues résistantes au 5-fluorouracile, ces souches pouvant être cultivées sur milieu industriel.

Lorsque le promoteur est celui du gène de la phéromone alpha, la levure sera de préférence de type sexuel Malalpha. On utilisera, par exemple, une souche de génotype ura3⁻ ou leu2⁻ ou autre, complémenté par le plasmide pour assurer le maintien du plasmide dans la levure par une pression de sélection appropriée.

Enfin, la présente invention concerne un procédé de préparation d'hirudine ou de l'un de ses variants par fermentation d'une levure transformée telle que décrite précédemment sur un milieu de culture et récupération de l'hirudine produite dans le milieu de culture sous forme mature ou sous forme d'un précurseur de l'hirudine maturable in vitro ou in vivo, ainsi que l'hirudine ou ses variants obtenus par ce procédé.

Ainsi, bien qu'il soit possible de préparer l'hirudine par fermentation des souches transformées précédentes sur un milieu de culture adapté, par accumulation d'hirudine dans les cellules, il est néanmoins préféré de faire sécréter l'hirudine dans le milieu, soit sous forme mature, soit sous forme d'un précurseur qui devra être processé in vitro.

Cette maturation peut se faire en plusieurs étapes. Tout d'abord, il peut être nécessaire de cliver certains éléments provenant de la traduction de la séquence Lₑₓ, ce clivage sera effectué au niveau de la séquence correspondant à S_{cl}. L'hirudine mature peut être précédée d'une méthionine qui sera coupée sélectivement par le bromure de cyanogène. Ce procédé est utilisable parce que la séquence codante de l'hirudine ne comprend pas de méthionine.

Il est également possible de prévoir à l'extrémité N le dipeptide Lys-Arg qui est coupé en COOH par une endopeptidase spécifique ; cette enzyme étant active dans le processus du sécrétion, on peut donc obtenir ainsi directement la protéine mature dans le milieu. Mais, dans certains cas, il peut être nécessaire de prévoir un clivage enzymatique après sécrétion en ajoutant une enzyme spécifique.

Dans certains cas, en particulier après un traitement au bromure de cyanogène, il peut être nécessaire de renaturer la protéine en recréant les ponts disulfures. Pour ce faire, le peptide est dénaturé, par exemple au chlorhydrate de guanidinium, puis renaturé en présence de glutathion réduit et oxydé.

Enfin, l'invention concerne l'hirudine obtenue par les procédés selon l'invention.

L'hirudine ainsi obtenue peut être utilisée à titre d'inhibiteur de la thrombine, comme médicament anticoagulant, comme produit de laboratoire pour empêcher la coagulation et/ou comme agent de diagnostic en utilisant le produit marqué.

En particulier, l'HV1 peut être utilisée dans des compositions pharmaceutiques, seule ou en combinaison avec d'autres principes actifs, ou bien dans le cadre de tests ou de diagnostic, in vitro ou in vivo. Dans ce dernier cas, il peut être intéressant de marquer la molécule, par exemple par un marquage radioactif, fluorescent, enzymatique ou autre.

Les exemples suivants illustreront d'autres caractéristiques et avantages de la présente invention.

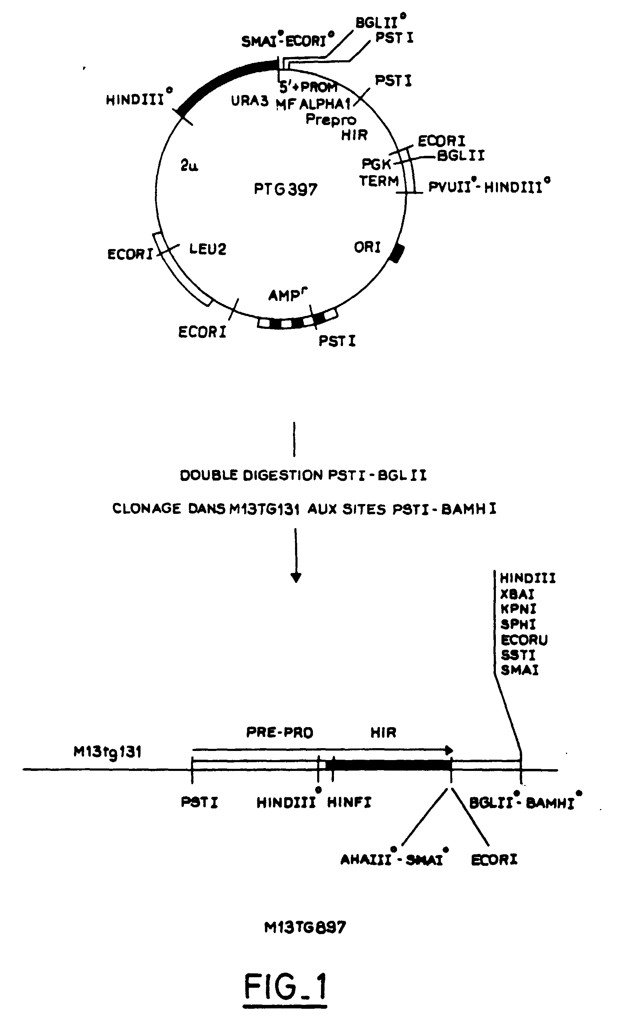

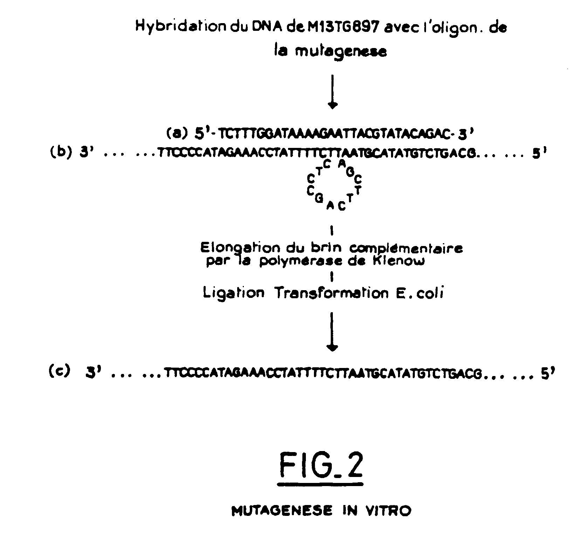

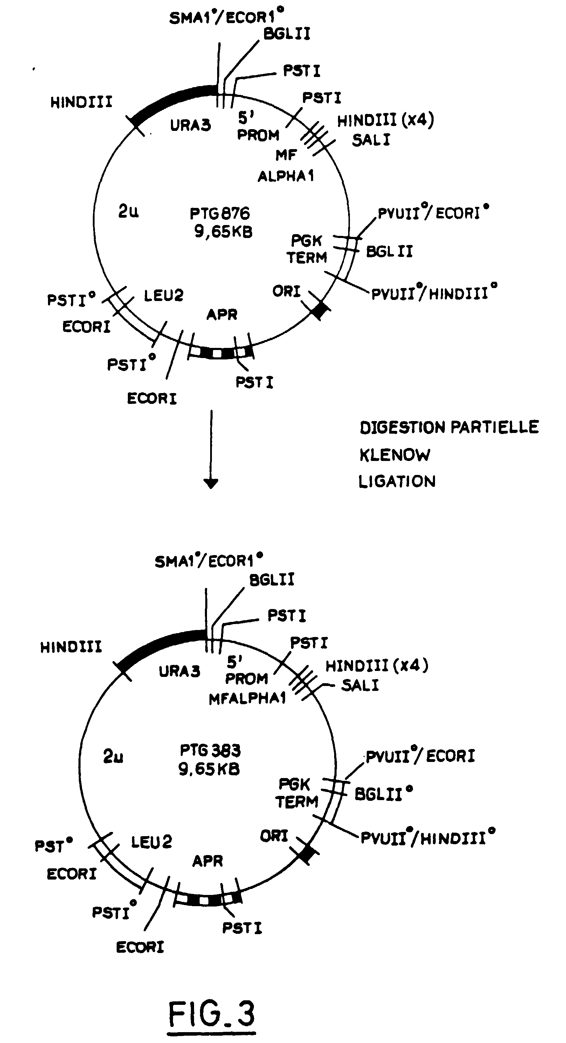

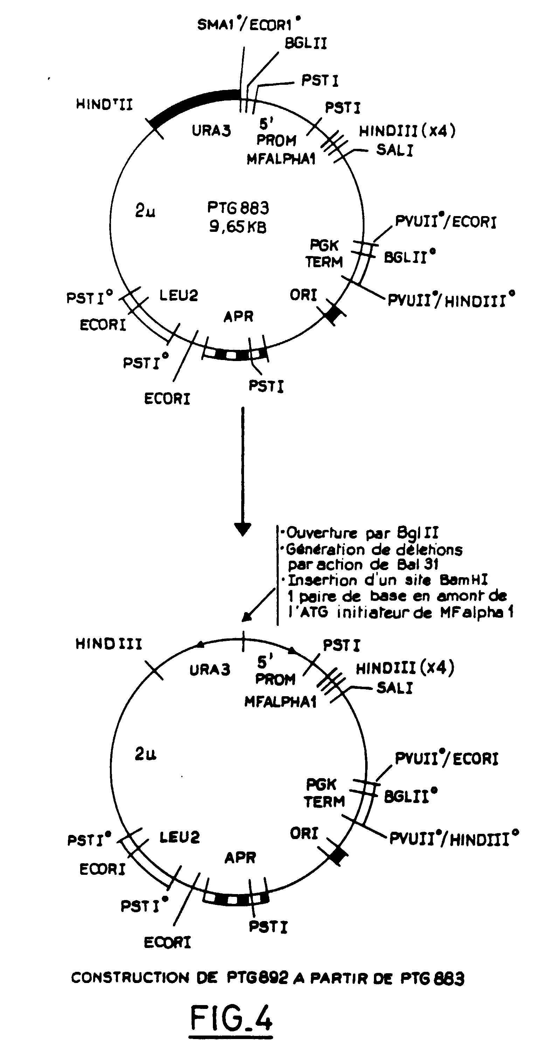

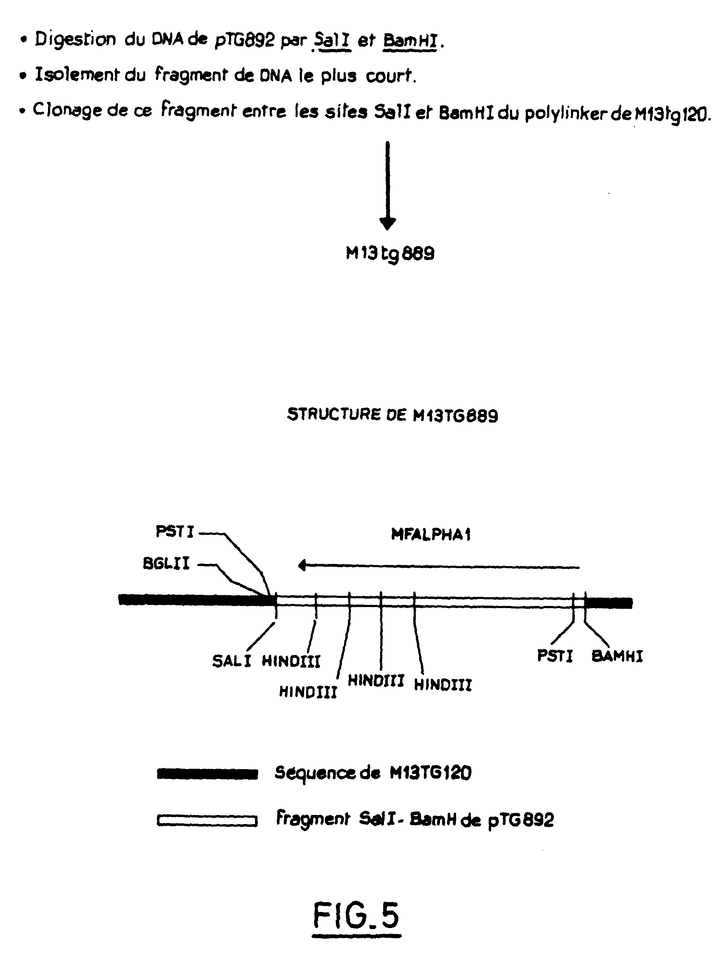

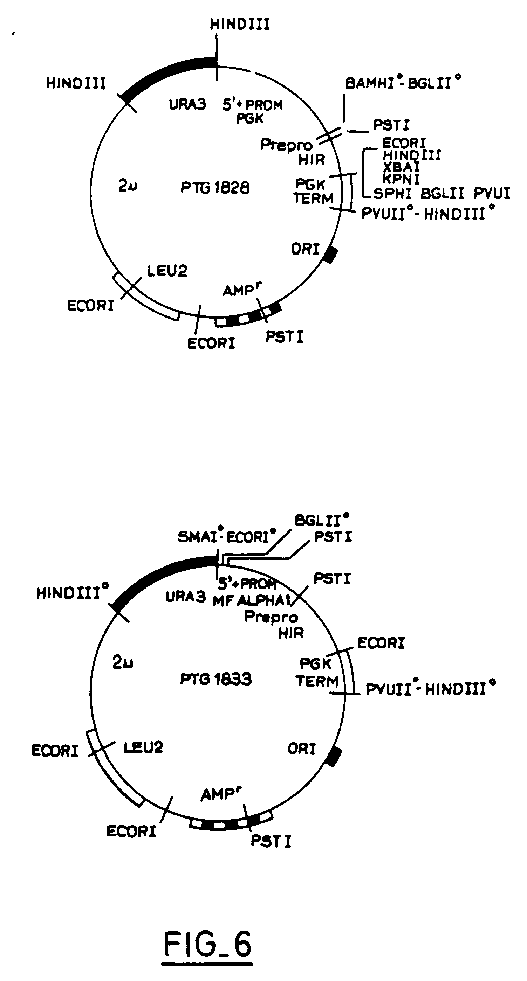

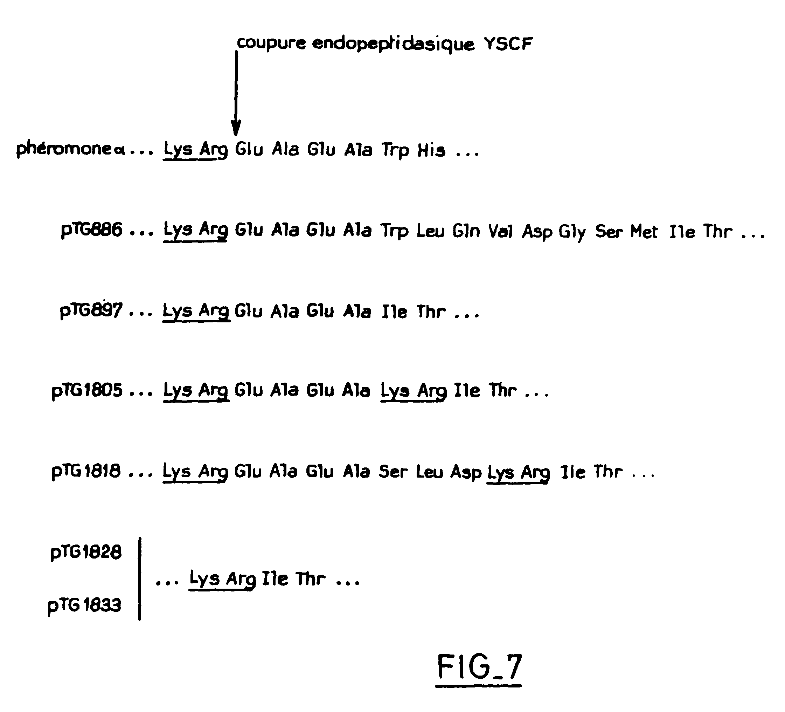

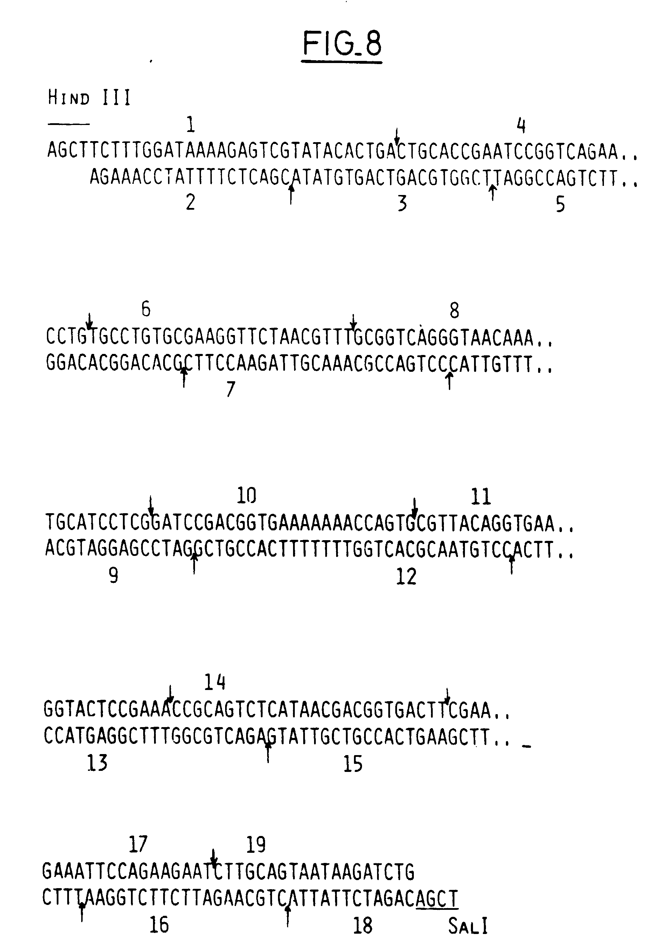

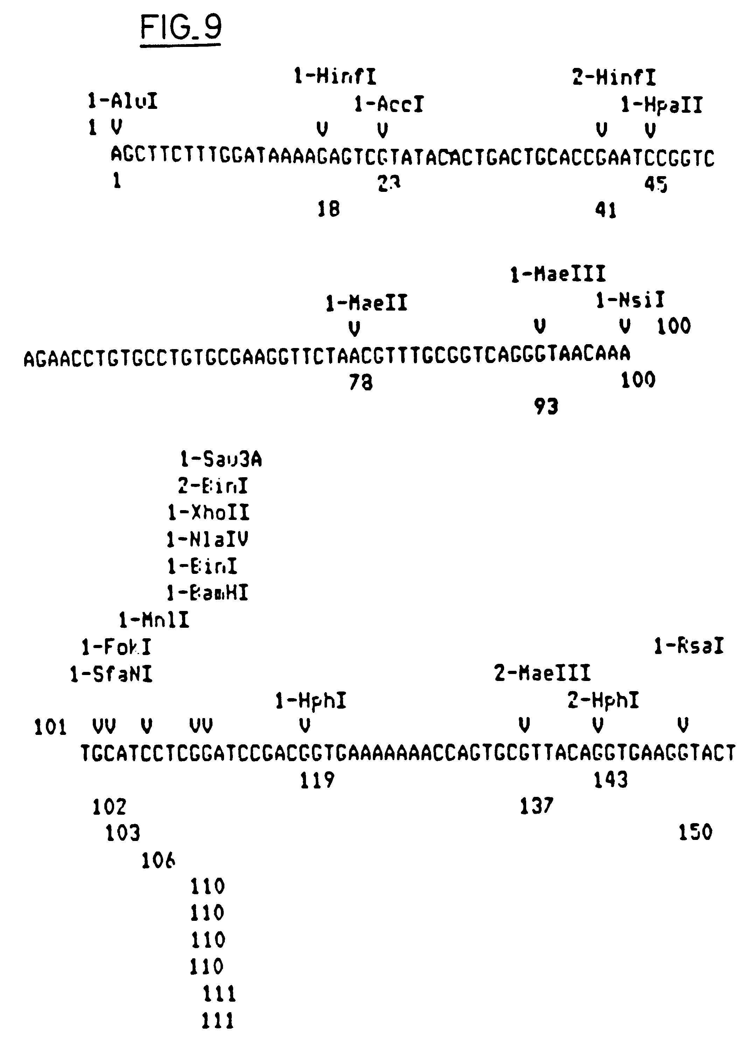

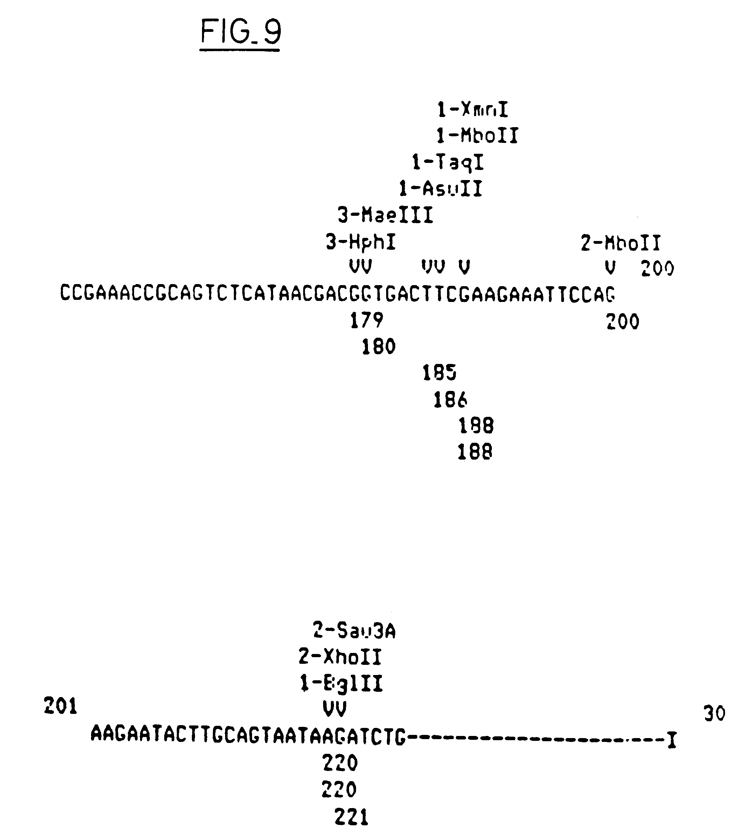

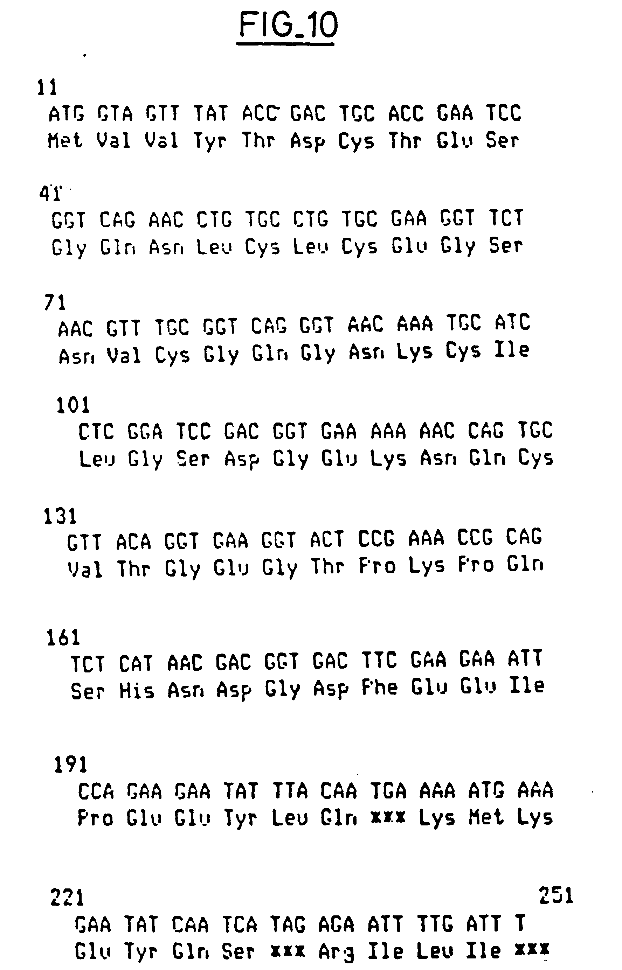

Sur les figures ci-annexées :
. la figure 1 représente la construction et la structure de M13TG897,
. la figure 2 représente la mutagénèse in vitro de l'ADN phagique de M13TG897 en M13TG1827 :
   a) séquence de l'oligonucléotide de la mutagénèse,
   b) séquence partielle de l'ADN de M13TG897 s'hybridant à l'oligonucléotide,
   c) séquence mutée,
. la figure 3 représente la construction de pTG883,
. la figure 4 représente la construction de pTG892,
. la figure 5 représente la construction de M13TG889,
. la figure 6 représente la structure de pTG1828 et pTG1833,
. la figure 7 représente la structure du précurseur de l'hirudine correspondant aux séquences portées par différents plasmides ; on a représenté la jonction entre a) le site de clivage (lys arg) le plus proche de l'extrémité NH₂ du précurseur et b) la séquence mature de l'hirudine HV2 (NH₂-Ile-Thr etc.).
. la figure 8 représente les séquences et la position des oligonuclétodies utilisés pour la synthèse de la séquence d'ADN codant pour HV1,
. les figures 9 et 10 représentent la carte de restriction et la traduction de la séquence en amino-acides lors de la synthèse de la séquence d'ADN codant pour HV1.

Les plasmides et stratégies utilisés et décrits dans la demande de brevet n^{o} 85 06672 ne sont pas repris dans la présente demande.

### EXEMPLE 1

Le plasmide pTG897 porte l'information pour un précurseur de l'hirudine qui ne contient qu'un seul site de clivage pour la protéinase yscF. La maturation à ce site de clivage libère une hirudine inactive. La coupure par la protéinase yscF libère une molécule d'hirudine allongée en NH₂ par 4 résidus Glu Ala Glu Ala. La délétion du fragment d'ADN codant pour cette séquence Glu Ala Glu Ala permettrait de libérer une chaîne d'hirudine correctement processée. Cette délétion a été réalisée par la technique de mutagénèse dirigée in vitro.

Le plasmide pTG897 a été découpé par double digestions PstI-BglII; le fragment d'ADN comprenant la séquence de l'hirudine a été cloné dans la forme réplicative du phage simple brin M13TG131 pour donner le phage M13TG897 (figure 1).

On a hybridé un oligonucléotide de séquence : 5'-TCTTTGGATAAAAGAATTACGTATACAGAC-3' à l'ADN génomique de ce phage M13TG897. Cet oligonucléotide s'hybride dans sa première moitié (15 paires de bases) avec la séquence de 15 paires de bases directement en amont de la séquence à déléter et dans sa seconde moitié avec la séquence de 15 paires de bases directement en aval (figure 2). Cet oligonucléotide a servi comme matrice pour la synthèse in vitro du brin complémentaire du génome du phage M13TG897. Après action de la T4 ADN ligase destinée à refermer de façon covalente le brin nouvellement formé, on transfecte une bactérie réceptrice, par exemple JM103 (Δ (Lac-Pro) SupE Thi EndlA sbcB15 strA rk- mk+/F' TraD36 ProAB+ LacI^{Q} LacZ Δ M15) et on analyse les phages issus de cette transfection par hybridation ADN-ADN en utilisant comme sonde l'oligonucléotide décrit précédemment et marqué au ³²P par kination, suivant les techniques habituelles pratiquées par l'homme de l'art (2). Cet ADN phagique correctement muté forme avec l'oligonucléotide de la mutagénèse un complexe d'hybridation repérable par sa plus grande stabilité, sa plus grande résistance aux conditions de stringence alors que le complexe d'hybridation formé par l'ADN phagique non muté et l'oligonucléotide est déstabilisé par la présence d'une boucle d'ADN simple brin.

### EXEMPLE 2

### Transfert du fragment muté dans un nouveau vecteur d'expression

Le plasmide pTG883 dérive du plasmide pTG876 par simple suppression du site BglII se trouvant près du terminateur de transcription du gène PGK.

Pour ce faire, on a digéré partiellement ce plasmide pTG876 par BglII, puis on a fait agir le fragment Klenow de l'ADN polymérase I de E. coli en présence des 4 nucléotides et on a lié covalemment les molécules d'ADN sur elles-mêmes grâce à l'action de l'ADN ligase du phage T4. Le plasmide pTG883 ne possède plus qu'un seul site BglII, situé en amont du promoteur du gène de la phéromone (figure 3).

Le plasmide pTG883 (20 µg) a été linéarisé par digestion BglII puis soumis à une digestion ménagée par la nucléase Ba131 (1.4 unités, 10 minutes - Boehringer Mannheim) suivant les techniques habituelles. L'ADN ainsi traité a été purifié par extractions successives au phénol et chloroforme - alcool isoamylique, puis une fraction (5 µg) a été soumise à l'action de la polymérase Klenow afin d'obtenir un maximum d'extrémités franches. L'ADN ainsi traité a été mis à liguer avec des linkers BamHI non phosphorylés (5'-CGGATCCCG) en présence de la ligase du phage T4 suivant la technique décrite par Lathe et al. (3). Après transformation de E-coli 1106 et sélection pour la résistance à l'ampicilline, on a isolé un plasmide pTG892 délété pour la région 5' flanquant le gène MFalphal (figure 4), le linker BamHI étant localisé une base en amont de l'ATG suivant le schéma :

5' - CGGGATCCCG   A   ATG AGA TTT ...
Linker BamHI   partie codante MTalpha1-2

Le nouveau fragment BamHI-SalI ainsi obtenu a été cloné entre les sites BamHI-SalI du phage M13TG120 pour donner le phage M13TG889 (figure 4). A partir du phage M13TG889, le fragment BamHI-BglII contenant toute la partie codante de la phéromone alpha a été isolé pour être ligué avec le grand fragment BglII du plasmide pTG848. Le plasmide obtenu après ligation, pTG892b, contient le fragment BamHI-BglII correspondant à la partie codante de la phéromone insérée derrière le site BglII du promoteur du gène PGK.

L'ADN de pTG892b comprend un site unique BglII, un site unique SalI, les deux sites enc adrent une courte séquence comprenant un site PstI. Par double digestion BglII et SalI, élution du grand fragment et ligation avec un polylinker synthétique, on a remplacé la courte séquence par le polylinker synthétique. Ce polylinker de séquence : 
5'-GATCCAGCTGACATCTGCATGCG
GTCGACTGTAGACGTACGCAGCT-5'
 présente une extrémité cohésive avec les fragments libérés par BglII, une extrémité cohésive avec les fragments libérés par SalI, ainsi que des sites de reconnaissance pour, entre autres, les enzymes PvuI, BglII et SphI.

Le nouveau vecteur ainsi obtenu a été appelé pTG1819. L'ADN de ce vecteur a été digéré par les enzymes SphI et PstI. Par cette double digestion, on obtient trois fragments : un PstI-PstI de 6,5 kb, un PstI-SphI de 1,9 kb et un autre SphI-PstI de 0,5 kb

Les deux fragments les plus longs ont été purifiés et mélangés avec le fragment SphI-PstI correspondant à l'hirudine mutée et provenant du phage M13TG1827, on a ainsi obtenu un nouveau vecteur d'expression : pTG1828 (figure 6).

### EXEMPLE 3

### Transfert du fragment dans le vecteur pTG881 : nouveau plasmide pTG1833

L'ADN de pTG881 a été partiellement digéré par PstI, de façon à libérer un fragment de 6,1 kb. Ce fragment a été isolé puis digéré totalement par BglII, ce qui libère 2 fragments PstI-PstI et PstI-BglII. Ces deux fragments ont été ligués avec le fragment PstI-BglII de 0,62 kb issu de pTG1828. On reconstitue ainsi un nouveau vecteur pTG1833 qui ne diffère du plasmide pTG897 que par la délétion des séquences codant pour Glu Ala Glu Ala. La structure du précurseur ainsi formé est présentée sur la figure 7.Ce plasmide pTG1833 possède une origine de réplication pour E. coli, un gène de résistance à l'ampicilline, le gène Leu2 de S. cerevisiae, un fragment du plasmide 2µ qui permet la réplication dans S. cerevisiae, le promoteur du gène MFalpha₁ codant pour pré-pro et le gène de l'hirudine (figure 6).

### EXEMPLE 4

### Transformation de la levure par les plasmides pTG1828 et pTG1833

On a transformé la souche S. cerevisiae TGYlsp4 (Matalpha ura3-251-373-328 his3-11-15) par les plasmides pTG1818, pTG1828 et pTG1833. Les résultats reportés dans le tableau 1 indiquent que pTG1818 et pTG1833 provoquent la sécrétion d'un même niveau d'activité hirudine. Par contre, TGYlsp4 pTG1828 sécrète deux fois moins d'activité hirudine que les deux autres souches. Comme contrôle, on a inclus une souche TGYlsp4 pTG881 qui ne possède pas l'information génétique pour l'hirudine. On a également comparé la production d'hirudine sécrétée par les souches de S. cerevisiae (Mat a)
TGY14-1 transformée soit par pTG1828 soit par pTG1833 et TGY14-2 transformée par les mêmes plasmides (tableau 2). Là encore, on remarque que dans le cas de la souche TGY14-2 (type sexuel Matalpha), le plasmide pTG1833 induit une meilleure sécrétion d'hirudine que le plasmide pTG1828. Par contre, dans le cas de la souche TGY14-1 (type sexuel Mat a) pTG1833 n'induit pas de sécrétion d'hirudine, comme attendu, alors que TGY14-2 produit l'hirudine.

**TABLEAU 1**

| Activité antithrombine des surnageants de cultures de levure (10 ml) (48 h de culture, milieu YNBG et casaminoacides 0,5 %) | | |
|---|---|---|
| Réceptrice | Plasmide | Activité totale |
| TGYlsp4 | pTG881 | non détectable |
| | pTG1818 | 158 |
| | pTG1828 | 73 |
| | pTG1833 | 149 |

**TABLEAU 2**

| Activité antithrombine des surnageants de culture de levure (10 ml) (72 h de culture, milieu YNBG + casaminoacides 0,5 %) | | |
|---|---|---|
| Réceptrice | Plasmide | Activité totale |
| TGYl4-1 | pTG881 | non détectable |
| | pTG1828 | 54 |
| | pTG1833 | non détectable |
| | | |
| TGYl4-2 | pTG881 | non détectable |
| | pTG1828 | 103 |
| | pTG1833 | 210 |

### EXEMPLE 5

### Isolement de souches pouvant être cultivées en milieu complet sans que le phénotype plasmidique soit perdu

On a montré dans le brevet WO 86/01224 que l'introduction de certaines mutations conférant la résistance au 5-FU dans une souche ura3 transformée en ura+ par le biais d'un plasmide permettait un plus grand choix de milieux pour la culture de ces souches transformées ; on pourraît en particulier utiliser les milieux complexes industriels. A partir de la souche TGY1sp4 pTG1833 on a isolé des mutants spontanés capables de former des colonies sur milieu complet (YPG) supplémenté avec du 5-Fluorouracile 1 mg/ml suivant le protocole décrit dans le brevet WO 86/01224. 6 mutants ont été cultivés en milieu complet et on en a choisi un qui ne présentait aucune colonie guérie pour le plasmide après plus de 20 générations de croissance en conditions non sélectives.

Le mutant, appelé TGY1sp4-3, peut être utilisé pour la production d'hirudine en milieu complet.

### EXEMPLE 6

### Stratégie de synthèse de la séquence d'ADN codant pour HV1

Cette séquence devra porter les éléments permettant la fusion en phase de la séquence d'HV1 avec la partie pre-pro de MFalphal et les signaux permettant une maturation correcte de l'hirudine sécrétée ; en particulier la région de jonction contiendra un doublet Lys-Arg juste en amont du premier résidu NH₂-terminal de HV1.

La stratégie utilisée est similaire à celle décrite dans le brevet français n^{o} 84 13250. La synthèse se fait en deux blocs séparés qui sont ensuite assemblés grâce à leurs extrémités cohésives BamHI. Le premier bloc comprend 9 oligonucléotides numérotés de 1 à 9 et le second bloc comprend 10 nucléotides numérotés de 10 à 19. Leurs séquences et la position des oligonucléotides sont représentés à la figure 8.

La carte de restriction ainsi que la traduction de la séquence en amino-acides sont données dans les figures 9 et 10.

### EXEMPLE 7

### Assemblage du gène synthétique

### .Premier bloc synthétique

Les oligonucléotides 2 à 8 (figure 8) ont été phosphorylés à leurs extrémités 5' pour éviter la formation de dimères ou de polymères : 500 picomoles de chaque oligonucléotide ont été traitées à la polynucléotide kinase (2 unités) dans un volume final de 25 µl de Tris HCl 60 mM, pH 7,5, MgCl₂ 10 mM, dithiothreitol 8 mM, contenant 3,3 pmoles de gamma-ATP ³²P (5 000 Ci/nmole) ; après 15 minutes d'incubation à 37°C on ajoute 5 nmoles d'ATP non marqué.

Après incubation à 37°C pendant 30 minutes, les fragments complémentaires ont été hybridés deux à deux, à l'exception des fragments 1, 2 et 3, ces derniers étant mélangés ensemble. 300 pmoles de chaque oligonucléotide ont été utilisés dans un volume final 10 µl de Tris HCl 66 mM, pH 7,5, MgCl₂ 6 mM, NaCl 100 mM, spermidine 0,5 mM, DTT 8 mM.

Ces mélanges chauffés à 100°C pendant 3 minutes sont ensuite refroidis lentement à 37°C pendant 2 heures. Les oligonucléotides 1-2-3 hybridés ont été mélangés avec les oligonucléotides 4-5 et incubés à 37°C pendant 2 heures. De la même façon, on a procédé à l'hybridation des couples des oligonucléotides 6-7 avec 8-9. Dans une dernière étape, on a rassemblé les 9 oligonucléotides ainsi prétraités, que l'on a incubés 2 heures à 37°C dans un volume final de 100 µl.

14 pmoles de ces oligonucléotides hybridés ont été soumis à un traitement par la ligase de T4 pendant 15 heures à 15°C. Ensuite on a ajouté au mélange réactionnel 50 ng du grand fragment HindIII-BamHI du M13TG131 préalablement purifié sur gel. Le mélange de ligation a ensuite été utilisé pour transformer des cellules compétentes d'E. coli JM103. Parmi 12 clones phagiques ainsi obtenus, un seul présentait la séquence recherchée : M13TG1888.

### . Deuxième bloc synthétique

On a utilisé la même stratégie pour synthétiser le second bloc (figure 8) qui a été cloné entre les sites BamHI et SalI du phage M13TG131. Deux clones sur les 12 testés ont présenté un insert correspondant à la séquence recherchée. Un de ces clones a été nommé M13TG1889.

### . Assemblage du gène synthétique

Les deux ADN double-brin de M13TG1888 et de M13TG1889 ont été digérés par HindIII et BamHI et par BamHI et SalI respectivement, et mélangés avec le grand fragment HindIII-SalI de pBR322 purifié sur gel. Après ligation, le mélange a servi à transformer E. coli 1106. Par sélection pour la résistance à l'ampicilline on a obtenu le plasmide pTG1890 qui contient la séquence synthétique correctement reconstituée.

### EXEMPLE 8

### Construction du vecteur pTG1891 qui permet la sécrétion de l'hirudine synthétisée

Le fragment d'ADN de pTG1890 qui porte la séquence codante de HV1 est repris sous forme de fragment HindIII-BglII (le site BglII étant situé juste en amont du site SalI qui a servi au clonage précédent) pour être inséré entre les sites HindIII et BglII du plasmide pTG881 décrit dans la demande de brevet n^{o} 85 06672. Dans le plasmide résultant, pTG1891, le gène HV1 se retrouve donc inséré entre les séquences pre-pro de MFalphal et le terminateur de transcription ; cette construction doit donc assurer la maturation et la sécrétion de l'hirudine HV1 puisqu'elle est identique à pTG1818 (brevet n^{o} 85 06672) à l'exception des séquences codantes de HV2 qui sont remplacées par celles de HV1.

### EXEMPLE 9

### Transformation d'une souche TGYlsp4 par l'ADN du plasmide pTG1891

La souche TGYlsp4 (alpha-1 ura3-251-373-328, His3-11-15) a été transformée par le plasmide pTG1891 ; 2 clones obtenus par sélection pour le caractère ura⁺ ont été mis en culture et leur poduction d'hirudine, sécrétée dans le milieu de culture, a été déterminée. Comme contrôle, on a dosé dans les mêmes conditions la quantité d'hirudine sécrétée par TGY1sp4 pTG1818.

On mesure deux fois plus d'activité antithrombine dans le surnageant de culture de la souche TGY1sp4 pTG1891 (0,9 mg/l de surnageant de culture) que dans celui de la souche TGYlsp4 pTG1833.

### Dépôt de souches représentatives de l'invention

Les souches suivantes ont été déposées à la Collection Nationale de Cultures de Microorganismes de l'Institut Pasteur, 28 rue du Docteur Roux, 75724 Paris Cédex 15 :
* le 6 juin 1986 :
   . Saccharomyces cerevisiae TGYlsp4 pTG1828, sous le n° I-569
   . Saccharomyces cerevisiae TGYlsp4.3pTG1833, sous le n° I-570.
* le 6 novembre 1986 :
   . Saccharomyces cerevisiae TGYlsp4 pTG1891, sous le n° I-623

### REFERENCES

1) Achstetter, T. et Wolf, D.H. (1985) EMBO Journal 4, 173.
2) Zoller, M.J. et Smith M.H. (1983) Meths in Enzym. 100, 469.
3) Lathe, R. ; Kieny, M.P. ; Skory, S. et Lecocq J-P (1984)DNA 3, 173.
4) Bussey H., Saville D., Greene D. et al. (1983) Mol. Cell. Biol. 3, 1362-1370.

## Revendications (Revendications pour l'(les) Etat(s) contractant(s) suivant(s): AT, BE, CH, LI, DE, FR, GB, IT, LU, NL, SE)

1. Bloc fonctionnel d'ADN permettant la préparation d'un variant d'hirudine (HV) à partir de levure, caractérisé en ce qu'il comporte:
- un premier fragment d'ADN codant pour un précurseur d'un variant d'hirudine; le dit précurseur comporte le dit variant hirudine ainsi qu'une région additionnelle comportant plus que deux amino-acides reliée par une liaison peptidique à l'extrémité N-terminale du dit variant hirudine ; et la dite région additionnelle comporte un seul site de clivage reconnu par la protéinase yscF, qui constitue la partie C-terminale de la dite région additionnelle,
- un second fragment d'ADN codant pour un peptide leader ; le dit second fragment d'ADN étant associé en phase à la partie 5' du dit premier fragment d'ADN,
- des régions de régulation appropriées pour l'expression des dits fragments d'ADN comportant un promoteur d'un gène de levure différent du promoteur du gène MF alpha 1.

2. Bloc fonctionnel d'ADN selon la revendication 1, dans lequel le premier fragment d'ADN code pour un précurseur d'un variant hirudine ; le dit précurseur comprenant le dit variant hirudine et une région additionnelle reliée par une liaison peptidique à l'extrémité N-terminale du dit variant hirudine ; et le dit précurseur étant caractérisé en ce que la région additionnelle comprend un site de clivage unique qui est le di peptide Lys-Arg.

3. Bloc fonctionnel d'ADN selon l'une des revendications 1 ou 2, dans lequel le premier fragment d'ADN code pour un précurseur du variant HV1 ou HV2.

4. Bloc fonctionnel d'ADN selon l'une des revendications 1 à 3, dans lequel le second fragment d'ADN code pour un peptide leader qui est le "pré" fragment du précurseur "pré-pro" du facteur alpha.

5. Bloc fonctionnel selon l'une des revendications 1 à 4, caractérisé en ce que le dit promoteur est le promoteur du gène PGK de levure.

6. Levure transformée par un bloc fonctionnel d'ADN selon l'une des revendications 1 à 5.

7. Levure selon la revendication 6, caractérisée en ce que le bloc fonctionnel d'ADN est inséré dans un plasmide qui comprend une origine de réplication fonctionnelle dans les levures.

8. Un plasmide qui comporte un bloc fonctionnel d'ADN selon l'une des revendications 1 à 5, et une origine de réplication fonctionnelle dans les levures.

9. Procédé de préparation d'un variant hirudine par fermentation d'une levure selon l'une des revendications 6 ou 7 et récupération du variant hirudine produit dans le surnageant du milieu de culture.

## Revendications (Revendications pour l'(les) Etat(s) contractant(s) suivant(s): ES, GR)

1. l'rocédé de préparation d'une levure caractérisée en ce que ladite levure est transformée par un bloc fonctionnel d'ADN permettant la préparation d'un variant d'hirudine (HV) à partir de levures comportant:
- un premier fragment d'ADN codant pour un précurseur d'un variant d'hirudine; le dit précurseur comporte le dit variant hirudine ainsi qu'une région additionnelle comportant plus que deux amino-acides reliée par une liaison peptidique à l'extrémité N-terminale du dit variant hirudine ; et la dite région additionnelle comporte un seul site de clivage reconnu par la protéinase yscF, qui constitue la partie C-terminale de la dite région additionnelle,
- un second fragment d'ADN codant pour un peptide leader ; le dit second fragment d'ADN étant associé en phase à la partie 5' du dit premier fragment d'ADN,
- des régions de régulation appropriées pour l'expression des dits fragments d'ADN comportant un promoteur d'un gène de levure différent du promoteur du gène MF alpha 1.

2. Procédé selon la revendication 1, dans lequel le premier fragment d'ADN code pour un précurseur d'un variant hirudine ; le dit précurseur comprenant le dit variant hirudine et une région additionnelle reliée par une liaison peptidique à l'extrémité N-terminale du dit variant hirudine ; et le dit précurseur étant caractérisé en ce que la région additionnelle comprend un site de clivage unique qui est le dipeptide Lys-Arg.

3. Procédé selon l'une des revendications 1 ou 2, dans lequel le premier fragment d'ADN code pour un précurseur du variant HV1 ou HV2.

4. Procédé selon l'une des revendications 1 à 3, dans lequel le second fragment d'ADN code pour un peptide leader qui est le "pré" fragment du précurseur "pré-pro" du facteur alpha.

5. Procédé selon l'une des revendications 1 à 4, caractérisé en ce que le dit promoteur est le promoteur du gène PGK de levure.

6. Procédé selon la revendication 5, caractérisée en ce que le bloc fonctionnel d'ADN est inséré dans un plasmide qui comprend une origine de réplication fonctionnelle dans les levures.

7. Procédé de préparation d'un plasmide, caractérisé en ce qu'on y intégre un bloc fonctionnel d'ADN tel que défini dans l'une des revendications 1 à 4, et une origine de réplication fonctionnelle dans les levures.

8. Procédé de préparation d'un variant hirudine par fermentation d'une levure obtenue par un procédé selon l'une des revendications 1 à 6 et récupération du variant hirudine produit dans le surnageant du milieu de culture.

## Patentansprüche (Patentansprüche für folgende(n) Vertragsstaat(en): AT, BE, CH, DE, FR, GB, IT, LI, LU, NL, SE)

1. Funktioneller DNA-Block, der die Herstellung einer Hirudin-Variante (HV) aus Hefe erlaubt, dadurch gekennzeichnet, daß er umfaßt:
- ein erstes DNA-Fragment, das für einen Vorläufer einer Hirudin-Variante codiert, wobei der genannte Vorläufer die genannte Hirudin-Variante sowie eine zusätzliche Region umfaßt, die mehr als zwei Aminosäuren enthält, die über eine Peptid-Bindung an das N-terminale Ende der genannten Hirudin-Variante gebunden sind, und die genannte zusätzliche Region eine einzelne Schnittstelle aufweist, die von der Proteinase yscF erkannt wird, die den C-terminalen Abschnitt der genannten zusätzlichen Region darstellt,
- ein zweites DNA-Fragment, das für ein Leader-Peptid codiert, wobei das genannte zweite DNA-Fragment in Phase an den 5'-Abschnitt des genannten ersten DNA-Fragments angelagert (assoziiert) ist, und
- Regulations-Regionen, die für die Expression der genannten DNA-Fragmente geeignet sind, die einen Promotor eines Hefe-Gens aufweisen, der von dem Promotor des Gens MF α1 verschieden ist.

2. Funktioneller DNA-Block nach Anspruch 1, bei dem das erste DNA-Fragment für einen Vorläufer einer Hirudin-Variante codiert, wobei der genannte Vorläufer die genannte Hirudin-Variante und eine zusätzliche Region umfaßt, die über eine Peptid-Bindung an das N-terminale Ende der genannten Hirudin-Variante gebunden ist, und der genannte Vorläufer dadurch gekennzeichnet ist, daß die zusätzliche Region eine einzelne Schnittstelle aufweist, bei der es sich um das Dipeptid Lys-Arg handelt.

3. Funktioneller DNA-Block nach einem der Ansprüche 1 oder 2, in dem das erste DNA-Fragment für einen Vorläufer der Variante HV1 oder HV2 codiert.

4. Funktioneller DNA-Block nach einem der Ansprüche 1 bis 3, in dem das zweite DNA-Fragment für ein Leader-Peptid codiert, bei dem es sich um das "Pre"-Fragment des Vorläufers "pre-pro" des Faktors α handelt.

5. Funktioneller DNA-Block nach einem der Ansprüche 1 bis 4, dadurch gekennzeichnet, daß es sich bei dem genannten Promotor um den Promotor des Gens PGK von Hefe handelt.

6. Hefe, die durch einen funktionellen DNA-Block nach einem der Ansprüche 1 bis 5 tranformiert worden ist.

7. Hefe nach Anspruch 6, dadurch gekennzeichnet, daß der funktionelle DNA-Block in ein Plasmid inseriert ist, das einen funktionellen Replikationsursprung in den Hefen umfaßt.

8. Plasmid, das einen funktionellen DNA-Block nach einem der Ansprüche 1 bis 5 und einen funktionellen Replikationsursprung in den Hefen umfaßt.

9. Verfahren zur Herstellung einer Hirudin-Variante durch Fermentation einer Hefe nach einem der Ansprüche 6 oder 7 und Gewinnung (Abtrennung) der in dem Kulturmedium-Überstand gebildeten Hirudin-Variante.

## Patentansprüche (Patentansprüche für folgende(n) Vertragsstaat(en): ES, GR)

1. Verfahren zur Herstellung einer Hefe, dadurch gekennzeichnet, daß die genannte Hefe durch einen funktionellen DNA-Block transformiert wird, der die Herstellung einer Hirudin-Variante (HV) aus Hefen erlaubt und der umfaßt:
- ein erstes DNA-Fragment, das für einen Vorläufer einer Hirudin-Variante codiert, wobei der genannte Vorläufer die genannte Hirudin-Variante sowie eine zusätzliche Region umfaßt, die mehr als zwei Aminosäuren enthält, die über eine Peptid-Bindung an das N-terminale Ende der genannten Hirudin-Variante gebunden sind, und die genannte zusätzliche Region eine einzelne Schnittstelle aufweist, die von der Proteinase yscF erkannt wird, die den C-terminalen Abschnitt der genannten zusätzlichen Region darstellt,
- ein zweites DNA-Fragment, das für ein Leader-Peptid codiert, wobei das genannte zweite DNA-Fragment in Phase an den 5'-Abschnitt des genannten ersten DNA-Fragments angelagert (assoziiert) ist, und
- Regulations-Regionen, die für die Expression der genannten DNA-Fragmente geeignet sind, die einen Promotor eines Hefe-Gens aufweisen, der von dem Promotor des Gens MF α1 verschieden ist.

2. Verfahren nach Anspruch 1, bei dem das erste DNA-Fragment für einen Vorläufer einer Hirudin-Variante codiert, wobei der genannte Vorläufer die genannte Hirudin-Variante und eine zusätzliche Region umfaßt, die über eine Peptid-Bindung an das N-terminale Ende der genannten Hirudin-Variante gebunden ist, und der genannte Vorläufer dadurch gekennzeichnet ist, daß die zusätzliche Region eine einzelne Schnittstelle aufweist, bei der es sich um das Dipeptid Lys-Arg handelt.

3. Verfahren nach einem der Ansprüche 1 oder 2, bei dem das erste DNA-Fragment für einen Vorläufer der Variante HV1 oder HV2 codiert.

4. Verfahren nach einem der Ansprüche 1 bis 3, bei dem das zweite DNA-Fragment für ein Leader-Peptid codiert, bei dem es sich um das "Pre"-Fragment des Vorläufers "pre-pro" des Faktors α handelt.

5. Verfahren nach einem der Ansprüche 1 bis 4, dadurch gekennzeichnet, daß es sich bei dem genannten Promotor um den Promotor des Gens PGK von Hefe handelt.

6. Verfahren nach Anspruch 5, dadurch gekennzeichnet, daß der funktionelle DNA-Block in ein Plasmid inseriert ist, das einen funktionellen Replikationsursprung in den Hefen umfaßt.

7. Verfahren zur Herstellung eines Plasmids, dadurch gekennzeichnet, daß man in dieses einen funktionellen DNA-Block einer Hefe nach einem der Ansprüche 1 bis 4 und einen funktionellen Replikationsursprung in den Hefen integriert.

8. Verfahren zur Herstellung einer Hirudin-Variante durch Fermentation einer Hefe, die nach einem Verfahren gemäß einem der Ansprüche 1 bis 6 erhalten worden ist, und durch Gewinnung (Abtrennung) der in dem Kulturmedium-Überstand gebildeten Hirudin-Variante.

## Claims (Claims for the following Contracting State(s): AT, BE, CH, DE, FR, GB, IT, LI, LU, NL, SE)

1. Functional DNA block enabling a hirudin variant (HV) to be prepared from yeast, characterized in that it contains:
- a first DNA fragment coding for a precursor of a hirudin variant; the said precursor contains the said hirudin variant as well as an additional region containing more than two amino acids linked via a peptide bond to the N-terminal end of the said hirudin variant; and the said additional region contains a single cleavage site recognized by the yscF proteinase, which constitutes the C-terminal portion of the said additional region,
- a second DNA fragment coding for a leader peptide; the said second DNA fragment being combined in frame with the 5' portion of the said first DNA fragment,
- regulatory regions suitable for the expression of the said DNA fragments, containing a promoter of a yeast gene different from the promoter of the MF alpha 1 gene.

2. Functional DNA block according to Claim 1, in which the first DNA fragment codes for a precursor of a hirudin variant; the said precursor comprising the said hirudin variant and an additional region linked via a peptide bond to the N-terminal end of the said hirudin variant; and the said precursor being characterized in that the additional region comprises a unique cleavage site which is the dipeptide Lys-Arg.

3. Functional DNA block according to either of Claims 1 and 2, in which the first DNA fragment codes for a precursor of the variant HV1 or HV2.

4. Functional DNA block according to one of Claims 1 to 3, in which the second DNA fragment codes for a leader peptide which is the "pre" fragment of the "prepro" precursor of the alpha factor.

5. Functional block according to one of Claims 1 to 4, characterized in that the said promoter is the promoter of the yeast PGK gene.

6. Yeast transformed by a functional DNA block according to one of Claims 1 to 5.

7. Yeast according to Claim 6, characterized in that the functional DNA block is inserted into a plasmid which comprises an origin of replication which is functional in yeasts.

8. A plasmid which contains a functional DNA block according to one of Claims 1 to 5, and an origin of replication which is functional in yeasts.

9. Method for preparing a hirudin variant by fermentation of a yeast according to either of Claims 6 and 7 and recovery of the hirudin variant produced in the supernatant of the culture medium.

## Claims (Claims for the following Contracting State(s): ES, GR)

1. Method for preparing a yeast, characterized in that the said yeast is transformed by a functional DNA block enabling a hirudin variant (HV) to be prepared from yeasts, containing:
- a first DNA fragment coding for a precursor of a hirudin variant; the said precursor contains the said hirudin variant as well as an additional region containing more than two amino acids linked via a peptide bond to the N-terminal end of the said hirudin variant; and the said additional region contains a single cleavage site recognized by the yscF proteinase, which constitutes the C-terminal portion of the said additional region,
- a second DNA fragment coding for a leader peptide; the said second DNA fragment being combined in frame with the 5' portion of the said first DNA fragment,
- regulatory regions suitable for the expression of the said DNA fragments, containing a promoter of a yeast gene different from the promoter of the MF alpha 1 gene.

2. Method according to Claim 1, in which the first DNA fragment codes for a precursor of a hirudin variant; the said precursor comprising the said hirudin variant and an additional region linked via a peptide bond to the N-terminal end of the said hirudin variant; and the said precursor being characterized in that the additional region comprises a unique cleavage site which is the dipeptide Lys-Arg.

3. Method according to either of Claims 1 and 2, in which the first DNA fragment codes for a precursor of the variant HV1 or HV2.

4. Method according to one of Claims 1 to 3, in which the second DNA fragment codes for a leader peptide which is the "pre" fragment of the "prepro" precursor of the alpha factor.

5. Method according to one of Claims 1 to 4, characterized in that the said promoter is the promoter of the yeast PGK gene.

6. Method according to Claim 5, characterized in that the functional DNA block is inserted into a plasmid which comprises an origin of replication which is functional in yeasts.

7. Method for preparing a plasmid, characterized in that a functional DNA block from a yeast according to one of Claims 1 to 4 and an origin of replication which is functional in yeasts are integrated therein.

8. Method for preparing a hirudin variant by fermentation of a yeast obtained by a method according to one of Claims 1 to 6 and recovery of the hirudin variant produced in the supernatant of the culture medium.
